Europäisches Patentamt

(19) **European Patent Office**

**Office européen des brevets**

(11) Veröffentlichungsnummer : **0 012 804**
**B2**

(12) # NEUE EUROPÄISCHE PATENTSCHRIFT

(45) Veröffentlichungstag der neuen Patentschrift :
**04.03.87**

(21) Anmeldenummer : 79104010.8

(22) Anmeldetag : 17.10.79

(51) Int. Cl.⁴ : **A 61 K 31/785**, A 61 K 31/19,
A 61 K 9/18, C 08 F 8/44,
C 07 C 59/68

(54) Salze eines zur dauernden Bindung an die Gallensäuren fähigen nicht toxischen aktivierten Anionenaustauscherharzes mit 2-(4'-(p-Chlorbenzoyl)-phenoxy)-2-(methyl)-propionsäure und diese Salze enthaltende Arzneimittel.

(30) Priorität : 21.12.78 IT 363178

(43) Veröffentlichungstag der Anmeldung :
**09.07.80 Patentblatt 80/14**

(45) Bekanntmachung des Hinweises auf die Patenterteilung : **30.12.81 Patentblatt 81/52**

(45) Bekanntmachung des Hinweises auf die Entscheidung über den Einspruch : **04.03.87 Patentblatt 87/10**

(84) Benannte Vertragsstaaten :
**AT BE CH DE FR GB NL SE**

(56) Entgegenhaltungen :
**DE-A- 1 492 245**
**DE-A- 2 151 510**
**DE-A- 2 427 924**
**GB-A- 857 193**
**GB-A- 857 194**
**GB-A- 1 099 269**
**US-A- 2 697 059**
**US-A- 2 990 332**
**Arzneimittelforschung 26 (1976) S. 885-894, 896-901**
**J. Pharm. Pharmacol. (1956) S. 975-986Int. Pharm. Abstr. 3 (1966) col. 956Dansk Tidsskr. Farm 40 (1966) S. 33-49, 55-88The Merck Index, 9. Auflage, S. 284, Nr. 2196Lancet 1975, S. 1083New England f. Med. 296 (1977), S. 1185-1190J. Lip. Res. 11 (1970) S. 404-411Eur. Journ. Clin. Invest. 9 (1979), S. 185-190Am. J.**

Pathol. 90 (1978) S. 435-445Proc. Nat. Acad. Sci. USA 73 (1976) S. 2043-2046"The disposition of orally administered cholestyramine C-14" New Jersey" (1965)J. Chromatogr. 1

(1976) S. 463-467J. Chromatogr. 145 (1978) S. 160-164J. Pharmacol. Exp. Ther. 178 (1971) S. 361-370J. Med. Chem. 19 (1976) S. 1214-1220Biochem. Pharmacol. 25 (1976) S. 2403-2406Atherosclerosis 27 (1977) S. 15-25Artery 4 (1978) S. 564-577

# 0 012 804

## Beschreibung

Die Erfindung betrifft neue Salze eines zur dauernden Bindung an die Gallensäuren fähigen nicht toxischen aktivierten Anionenaustauscherharzes mit 2-[4'-(p-Chlorbenzoyl)-phenoxyl]-2-[methyl]-propionsäure und diese Salze enthaltende Arzneimittel, insbesondere zur Verminderung des Cholesterin- und Triglyceridgehaltes im Blut.

Es herrscht die Auffassung, dass das Entstehen von vielen Krankheiten des Herz/Kreislauf-Systemes unmittelbar mit hohen Triglycerid- und Cholesteringehalten im Blut zusammenhängt. Daher wurden zahlreiche zur Verminderung von erhöhten Cholesterin- und Triglyceridgehalten im Blut unter Zurückbringen derselben auf die normalen Werte fähige Arzneimittel vorgeschlagen.

Unter diesen Arzneimitteln sind Produkte, deren Struktur auf die der [Phenoxy]-[methyl]propionsäure zurückgeführt werden kann, bekannt und verbreitet im Handel. Insbesondere sind der 2-[p-Chlorphenoxy]-2-[methyl]-propionsäureäthylester (Clofibrat) und 2-[4'-(p-Chlorbenzoyl)-phenoxy]-2-[methyl]-propionsäureisopropylester (Procetophen) weit verbreitet.

In der therapeutischen Praxis werden auch starke Anionenaustauscherharze (wie Mischpolymerisate aus Styrol und etwa 2 Gew.-% Divinylbenzol, die als aktive Gruppen quaternäre Ammoniumgruppen enthalten Colestyramin und Colestipol), welche bei Verabreichung in Form von wässrigen Suspensionen indirekt die Verminderung des Blutcholesteringehaltes durch einen Mechanismus, der die Immobilisierung der im Darmtrakt des Verdauungssystemes vorliegenden Gallensäuren unter Bildung von unlöslichen und irreversiblen Komplexen, welche schliesslich mit den Exkrementen ausgeschieden werden, umfasst, begünstigen, verwendet. In den britischen Patentschriften 929 391 und 1 286 949 sind solche Harze und ihre therapeutische Anwendung beschrieben.

Aus der deutschen Offenlegungsschrift 2 151 510 sind auch mechanische Mischungen von nicht toxischen starken Anionaustauscherharzen mit Verbindungen der oben genannten Art [2-(p-Chlorphenoxy)-2-(methyl)-propionsäure {mit anderer Bezeichnung α-(p-Chlorphenoxy)-isobuttersäure} sowie deren Natriumsalz und Estern, wie 2-(p-Chlorphenoxy)-2-(methyl)-propionsäureäthylester] bekannt. Solche Mischungen haben meistens eine höhere cholesterinsenkende Wirksamkeit als die Eigenwirksamkeiten ihrer 2 Bestandteile. Zum Nachweis der Tatsache, dass es sich in der genannten Druckschrift um mechanische Mischungen der Anionenaustauscherharze mit den genannten Verbindungen und nicht um Salze aus ihnen handelt, wurden nach dem Beispiel 8 der deutschen Offenlegungsschrift 2 151 510 DEAE Dextranhydrochlorid und Natrium-p-chlorphenoxyisobutyrat in Wasser vermischt, wodurch eine Mischung, in welcher nahezu das gesamte Natriumsalz der p-Chlorphenoxyisobutyrat in Wasser vermischt, wodurch eine Mischung, in welcher nahezu das gesamte Natriumsalz der p-Chlorphenoxyisobuttersäure als solches unverändert blieb, erhalten wurde, was durch spektrophotometrische UV-Analyse ($\alpha$ = 278 nm) der in der filtrierten wässrigen Lösung verbliebenen freien p-Chlorphenoxyisobuttersäure nachgewiesen wurde. Aus dieser Druckschrift sind weder Mischungen noch Salze der 2-[4'-(p-Chlorbenzoyl)- phenoxy]-2-[methyl]-propionsäure bekannt.

Ferner sind aus der deutschen Offenlegungsschrift 24 27 924 Salze von nicht verzweigten und nicht vernetzten linearen Polymeren mit zur Salzbildung befähigten und durch Alkylenreste aneinander .gebundenen Stickstoffatomen unter anderem mit 2-(p-Chlorphenoxy)-2-methylpropionsäure zum Binden von Gallensäuren im Magen/Darm-Trakt bekannt. Bei den Polymeren dieser Salze handelt es sich um nicht verzweigte und nicht vernetzte Polymere, wie es in der genannten Druckschrift hervorgehoben wird (vergleiche insbesondere Seite 4, Zeile 17 bis 18, Seite 9, Zeile 1 bis 20 und Seite 11, Zeile 9 bis 10 derselben). Auch nehmen bei den Polymeren der Salze der deutschen Offenlegungsschrift 24 27 924 die Stickstoffatome an der Polymerisationsreaktion zu ihrer Herstellung teil und daher beteiligen sie sich auch an der Polymerkette unter Bildung eines Teiles derselben. Anders ausgedrückt handelt es sich um Stickstoffpolymere (vergleiche Seite 8, Zeile 21 bis 22 der genannten Druckschrift).

Weiterhin sind in der britischen Patentschrift 857 194 Salze von Anionenaustauscherharzen, wie Polymeren von Styrol mit freien sauren oder basischen Gruppen oder mit mit Divinylbenzol vernetzter polymerisierter Methacrylsäure mit freien Carboxylgruppen, mit Säuren, von welchen keine ein [Phenoxy]-[methyl]-propionsäurederivat ist, beschrieben. Diese Harze dienen aber nur als Träger von physiologisch aktiven Bestandteilen, wobei keine Angabe über eine spezifische Indikation gemacht ist.

Schliesslich sind aus der britischen Patentschrift 1 099 269 « Assoziationen » von p-Chlorphenoxyisobuttersäurederivaten mit natürlichem Heparinoid zur Verminderung des Cholesterinspiegels im Blut bekannt. Diese « Assoziationen » sind jedoch keine Salze, sondern einfache Mischungen, wie es aus den Beispielen und der Tatsache, dass die « Assoziationen » nicht solche der freien p-Chlorphenoxyisobuttersäure selbst, sondern von Estern oder Salzen mit Kationen sind, hervorgeht.

Der Erfindung liegt die Aufgabe zugrunde, überlegene pharmakologische, insbesondere den Cholesterin- und Triglyceridgehalt im Blut senkende, neue Salze eines Anionenaustauscherharzes der oben erwähnten Art und solche enthaltende Arzneimittel zu schaffen.

Es wurde nun überraschenderweise festgestellt, dass ein nicht toxisches Anionenaustauscherharz, welches mit 2-[4'-(p-Chlorbenzoyl)-phenoxy]-2-[methyl]-propionsäure in Salze überführt ist, hinsichtlich der Verminderung der im Blut in übermässigen Mengen vorliegenden Cholesterin und Lipide (Fette und fettähnlichen Substanzen) unter drastischer Verminderung derselben auf die normalen Werte überlegen

3

wirksam ist.

Gegenstand der Erfindung sind daher Salze eines zur dauernden Bindung an die Gallensäuren fähigen nicht toxischen aktivierten Anionenaustauscherharzes, nämlich eines mit 2 Gew.-% Divinylbenzol vernetzten Polystyrolharzes mit Aminogruppen in der Ammoniumchloridsalzform mit 2-[4'-(p-Chlorbenzoyl)-phenoxy]-2-[methyl]-propionsäure, bei welchen die Ionenaustauschkapazität des Harzes zu 1,99 bis 15 % als Salz vorliegt.

Durch das bei der Erörterung der deutschen Offenlegungsschrift 2 151 510 angegebene Verfahren ergab sich gegenüber dieser Druckschrift bei den erfindungsgemässen Salzen der Prozentgehalt am Natriumsalz der freien 2-[4'-(p-Chlorbenzoyl)-phenoxy]-2-[methyl]-propionsäure in der Lösung zu 0. Dies ist auf die Tatsache zurückzuführen, dass nur durch Perkolieren beziehungsweise Giessen einer Lösung des Natriumsalzes der betreffenden Säure auf ein aktiviertes Anionenaustauscherharz, wie unter den weiter unten folgenden Punkten I) und II) ein vollständiger Salzbildung erhalten wird.

Die therapeutische Wirksamkeit der erfindungsgemässen Salze ist überraschenderweise viel höher als die der aus der deutschen Offenlegungsschrift 2 151 510 bekannten Mischungen.

Auch gegenüber dem übrigen Stand der Technik ist die Erfindung überraschend. So wird in der deutschen Offenlegungsschrift 24 27 924 von verzweigten und vernetzten Harzen als Harzen der Salze geradezu abgeraten (vergleiche insbesondere Seite 9, Zeile 1 bis 20 und Seite 11, Zeile 9 bis 10 derselben), wobei sogar angegeben ist, dass bei ihrer Herstellung die Verzweigung und Vernetzung zu verhindern ist. Demgegenüber ist das Harz der erfindungsgemässen Salze verzweigt und vernetzt. Auch beteiligen sich beim Harz der erfindungsgemässen Salze die Stickstoffatome nicht an der Polymerkette beziehungsweise am Polymernetz oder anders ausgedrückt am Polymerskelett, sie sind vielmehr an Kohlenstoffatome desselben gebunden. Es ist auch noch zu bemerken, dass für die Salze der deutschen Offenlegungsschrift 24 27 924 keine 2-[4'-(p-Chlorbenzoyl)-phenoxy]-2-[methyl]-propionsäure der erfindungsgemässen Salze verwendet wird. Auch in der britischen Patentschrift 857 194 ist von keinem 2-[4'-(p-Chlorbenzoyl)- phenoxy]-2-[methyl]-propionsäurederivat die Rede, während in der britischen Patentschrift 1 099 269 kein unter die obige erfindungsgemässe Definition fallendes Harz vorgesehen ist.

Das Polymerskelett des genannten Anionenaustauscherharzes kann durch Polymerisation von Styrolmonomer in Gegenwart von 2 Gew.-% Divinylbenzol als Vernetzungsmittel in bekannter Weise erhalten worden sein.

Gegenstand der Erfindung sind auch Arzneimittel, welche eines oder mehrere erfindungsgemässe Salze als Wirkstoff beziehungsweise Wirkstoffe, gegebenenfalls zusammen mit einem oder mehreren pharmazeutischen Konfektionierungsmitteln, enthalten.

Die erfindungsgemässen Arzneimittel liegen vorteilhaft in Arzneimittelpräparaten zur peroralen Verabreichung vor. Die tägliche wirksame Mindestdosis der erfindungsgemässen Salze bei Menschen ist etwa 10 g. Es ist jedoch in Anbetracht ihrer niedrigen Toxizität möglich, auch höhere Tagesdosen zu verabreichen. Die bevorzugte Dosis beträgt 12 bis 30 g.

Die erfindungsgemässen Arzneimittel können in Form von Arzneimittelpräparaten herkömmlicher Art, wie Sirupen und Suspensionen, gegebenenfalls mit einem Gehalt an Hilfsstoffen, wie Excipienten beziehungsweise Trägern, Konservierungsmitteln, Gleitmitteln, Stabilisatoren, Netzmitteln, Emulgiermitteln, Salzen zur Änderung des osmotischen Druckes, Puffern, Farbstoffen, Aromastoffen und/oder Süssungsmitteln, vorliegen.

Die erfindungsgemässen Salze können wie folgt hergestellt werden.

I) Aktivierung des Harzes

Das Anionenaustauscherharz wird aufeinanderfolgend mit einer wässrigen 0,1 n Salzsäure, destilliertem Wasser und einer 0,1 n Natriumhydroxydlösung gewaschen. Das Waschen wird durch Suspendieren des Harzes in der Waschflüssigkeit, Dekantieren des Harzes und Entfernen der überstehenden Flüssigkeit bewerkstelligt. Die Waschfolge wird 3-mal wiederholt. Das Harz wird noch mit einer 0,1 n Salzsäure und danach mit destilliertem Wasser bis zur Erreichung der neutralen Reaktion gewaschen.

Das gewaschene Harz wird in feuchten Zustand bis zum Zeitpunkt seines Gebrauches aufbewahrt.

Die Aktivierung kann auch mit anderen Säuren als Salzsäure durchgeführt werden.

II) Überführung des Harzes in das Salz

Es wird eine Chromatographierkolonne mit einem Durchmesser von 1 cm bis zu einer Höhe von 2 cm mit dem wie unter dem vorstehenden Abschnitt I) beschrieben gewaschenen Anionenaustauscherharz gefüllt.

Aus den Harzvolumen und der spezifischen Austauschkapazität des Harzes selbst lässt sich die Höchstaustauschkapazität errechnen.

Die mit dem Harz in ein Salz zu überführende Säure wird in der äquivalenten Menge einer n Natriumhydroxydlösung gelöst und die Lösung wird in die Kolonne gegossen. Es wird mit destilliertem Wasser bis zum Verschwinden der Anionen eluiert.

Das so in ein Salz überführte Anionenaustauscherharz kann entweder in der nassen Form, wie es bei

**0 012 804**

der Salzbildungsreaktion erhalten wurde, verwendet werden oder zur Erzielung einer Suspension verdünnt werden oder getrocknet und im trockenen Zustand aufbewahrt werden.

Der Grad der Überführung in die Salzform kann wie folgt bestimmt werden :

III) Bestimmung des Grades der Salzbildung der basischen Gruppen des Anionenaustauscherharzes

Es wird eine Suspension des wie unter dem obigen Abschnitt I) beschrieben aktivierten Anionenaustauscherharzes in Wasser in eine Chromatographierkolonne eingeführt und mit einer Lösung der Säure, welche mit dem Harz in ein Salz zu überführen ist, in der äquivalenten Menge einer Natriumhydroxydlösung behandelt.

Anschliessend wird mit Wasser eluiert, wobei das ganze Eluat gesammelt wird. Das im Eluat vorliegende Anion wird titriert und aus dem Verhältnis des so gefundenen Wertes zum ursprünglich im Harz vorliegenden Anionengehalt kann die prozentuale Salzbildung errechnet werden.

Die Prüfung der pharmakologischen Wirkung der erfindungsgemässen Salze kann wie folgt erfolgen :

A) Bestimmung der Fähigkeit zur Verminderung des Triglyceridgehaltes im Serum von Ratten bei durch Fruchtzucker (Fructose) herbeigeführtem übermässigen Triglyceridgehalt (Hypertriglyceridämie)

Es wurden normal gefütterte männliche Wistar-Ratten mit Gewichten von 250 ± 20 g in Gruppen von je 6 Tieren unterteilt. Die in Propylenglykol gelösten oder in Wasser suspendierten Substanzen wurden peroral mittels einer Magensonde in einer Dosis von 0,05 Millimoll Säure/kg Körpergewicht in 5 cm$^3$ Lösungsmittel oder Suspendiermittel/kg Körpergewicht verabreicht.

Die Tiere wurden 3 Tage lang mit dem zu untersuchenden Produkt behandelt. Am ersten Tag wurden sie ohne Trinkwasser gelassen, während ihnen an den beiden aufeinanderfolgenden Tagen eine 20 %ige Fruchtzuckerlösung zum Trinken angeboten wurde. Die Tiere wurden in Ätheranästhesie geopfert und ihr Blut wurde mittels intrakardialer Punktion entnommen. Im Serum wurden die Triglyceride durch enzymatische Dosierung gemäss M. Eggstein, F.H. Kreutz und F.H. Schmidt (M. Eggstein und F.H. Kreutz, Klin. Wschr., 44 [1966], 262 ; F.H. Schmidt und K. Von Dahl, Z. Klin. Chem. 6 [1968], 156) ermittelt.

B) Bestimmung der Änderung des Cholesteringehaltes im Blut und in der Leber von Ratten

Es wurden normal gefütterte männliche Wistar-Ratten mit Gewichten von 280 ± 20 g in Gruppen von je 6 Tieren unterteilt. Die zu untersuchenden Substanzen wurden in Lösung in Propylenglykol oder in Suspension in Wasser peroral mittels einer Magensonde in einer Dosis von 0,6 Millimol Säure/kg Körpergewicht in 5 cm$^3$ Lösungsmittel oder Suspendiermittel/kg Körpergewicht verabreicht.

Die Tiere wurden zu den bestimmten Zeiten in Ätheranästhesie geopfert und ihr Blut wurde mittels intrakardialer Punktion entnommen. Im Serum wurde der Cholesteringehalt bestimmt. Gleichzeitig wurde auch das Lebercholesterin bestimmt. Die Dosierung zur Bestimmung des Cholesterines wurde nach D. Watson (Clin. Chim. Acta 5 [1960], 637) durchgeführt.

C) Bestimmung der Änderung der Gesamtlipide, des Gesamtcholesterines und der Triglyceride bei Ratten, welche mit einer hypercholesterinämischen Diät von Greenberg mit der Abwandlung nach Tensho und Mitarbeitern gefüttert worden sind

Es wurden männliche Wistar-Ratten mit Gewichten von 90 bis 100 g 7 Tage lang mit der hypercholesterinämischen Diät von Tensho und Mitarbeitern (Acutely induced hypercholesterolemic in the rat — Yakugaku Zasshi 1972, 92, 879) gefüttert. Während der letzten 3 Tage wurden sie peroral 2-mal täglich mit 650 mg/kg erfindungsgemässen Verbindungen beziehungsweise Vergleichsmaterialien behandelt.

Nach 17 Stunden langer Nüchternheit wurden die Gesamtlipid-, Gesamtcholesterin- und Triglyceridspiegel im Blut nach den Verfahrensweisen von D. Watson, Clin. Chim. Acta 5 [1960], 637, M. Eggstein, Klin. Wschr. 44 [1966], 267 und N. Zoellner und K. Kirsch Z. gss. exp. Med. 135 [1962], 545 bestimmt.

Nach der unter dem obigen Abschnitt A) beschriebenen Verfahrensweise wurde die Fähigkeit eines erfindungsgemässen Salzes sowie des als Vergleichsprodukt verwendeten entsprechenden Anionenaustauscherharzes und des ebenfalls als Vergleichsprodukt verwendeten Natriumsalzes der 2-[4'-(p-Chlorbenzoyl)-phenoxy]-2-[methyl]-propionsäure und der weiterhin als Vergleichsprodukt verwendeten mechanischen Mischung der beiden letzten, jeweils mit denselben Mengen dieser beiden Substanzen, in welchen sie im erfindungsgemässen Salz enthalten waren, zur Verminderung des Triglyceridgehaltes bei Ratten mit durch Fruchtzucker herbeigeführtem übermässigem Triglyceridgehalt (Hypertriglyceridämie) bestimmt. Die prozentualen Änderungen des Triglyceridgehaltes im Blut, bezogen auf die nicht behandelten Blindversuchstiere, welche nur mit Fruchtzucker behandelt worden sind, sind in der folgenden Tabelle I zusammengestellt.

Tabelle I

| Produkt | Tagesdosis | Änderung des Triglyceridgehaltes im Blut, bezogen auf den Blindversuch |
| --- | --- | --- |
| Salz eines mit 2 Gew.-% Divinylbenzol vernetzten Polystyrolharzes mit Aminogruppen in der quaternären Ammoniumchloridsalzform (Dowex 1X2) mit | 616 mg/kg | − 79% |

5

Fortsetzung der Tabelle I

| Produkt | Tagesdosis | Änderung des Triglycerid-gehaltes im Blut, bezogen auf den Blindversuch |
|---|---|---|
| 2-[4'-(p-Chlorbenzoyl)-phenoxy]-2-[methyl]-propion-säure, dessen Ionenaustauschkapazität zu 2,38% ge-sättigt ist [erfindungsgemässes Salz des Beispieles 2] | | |
| Das obige mit 2% Divinylbenzol vernetzte Polystyrol-harz mit Aminogruppen in der quaternären Ammo-niumchloridsalzform (Dowex 1X2) [Vergleichspro-dukt] | 600 mg/kg | − 18% |
| Natriumsalz der 2-[4'-(p-Chlorbenzoyl)-phenoxy]-2-[methyl]-propionsäure [Vergleichsprodukt] | 15,9 mg (=0,05 Millimol)/kg | − 63% |
| Mechanische Mischung aus 600 mg des obigen mit 2% Divinylbenzol vernetzten Polystyrolharzes mit Aminogruppen in der quaternären Ammoniumchlo-ridsalzform (Dowex 1X2) und 15,9 mg des Natrium-salzes der 2-[4'-(p-Chlorbenzoyl)-phenoxy]-2-[me-thyl]-propionsäure [Vergleichsprodukt ] | 616 mg/kg | − 57% |

Es wurde auch die Fähigkeit des für die vorstehenden Versuche verwendeten erfindungsgemässen Salzes und der für die vorstehenden Versuche verwendeten Vergleichsprodukte zur Wirkung auf den Cholesteringehalt im Blut und in der Leber von Ratten nach der unter dem obigen Abschnitt B) beschriebenen Verfahrensweise bestimmt. Die prozentualen Änderungen des Cholesteringehaltes zu verschiedenen Zeiten, bezogen auf die nicht behandelten Blindversuchstiere, sind in der folgenden Tabelle II zusammengestellt.

Tabelle II

| Produkt | Tagesdosis | Änderung des Cholesteringehaltes, bezogen auf den Blindversuch | | | | | |
|---|---|---|---|---|---|---|---|
| | | in der Leber | | | im Blut | | |
| | | nach 7 Stunden | nach 24 Stunden | nach 48 Stunden | nach 7 Stunden | nach 24 Stunden | nach 48 Stunden |
| Salz eines mit 2 Gew.-% Divinylbenzol ver-netzten Polystyrolhar-zes mit Aminogruppen in der quaternären Ammoniumchlorid-salzform (Dowex 1X2) mit 2-[4'-(p-Chlorben-zoyl)-phenoxy]-2-[me-thyl]-propionsäure, dessen Ionenaus-tauschkapazität zu 2,38% gesättigt ist [er-findungsgemässes Salz des Beispieles 2] | 800 mg/kg | − 16% | − 15% | − 11% | +8% | − 43% | − 36% |
| Das obige mit 2% Divi-nylbenzol vernetzte Polystyrolharz mit Aminogruppen in der quaternären Ammo-niumchloridsalzform (Dowex 1X2) [Ver-gleichsprodukt] | 600 mg/kg | ÷6% | − 3% | − 7% | +9% | ÷7% | − 29% |
| Natriumsalz der 2-[4'-(p-Chlorbenzoyl)-phen-oxy]-2-[methyl]-pro-pionsäure [Vergleichs-produkt] | 200 mg/kg (= =0,6 Milli-mol/kg) | − 17% | ÷25% | − 7% | 0% | − 37% | − 26% |

Fortsetzung der Tabelle II

| Produkt | Tagesdosis | Änderung des Cholesteringehaltes, bezogen auf den Blindversuch | | | | | |
|---------|-----------|:---:|:---:|:---:|:---:|:---:|:---:|
| | | in der Leber | | | im Blut | | |
| | | nach 7 Stunden | nach 24 Stunden | nach 48 Stunden | nach 7 Stunden | nach 24 Stunden | nach 48 Stunden |
| Mechanische Mischung aus 600 mg des obigen mit 2% Divinylbenzol vernetzten Polystyrolharzes mit Aminogruppen in der quaternären Ammoniumchloridsalzform (Dowex 1X2) und 200 mg des Natriumsalzes der 2-[4'-(p-Chlorbenzoyl)-phenoxy]-2-[methyl]-propionsäure [Vergleichsprodukt] | 800 mg/kg | − 4% | ÷7% | − 23% | − 11% | − 33% | − 15% |

Aus den obigen Tabellen I und II geht hervor, dass das erfindungsgemässe Salz sich gegenüber dem genannten verwendeten Anionenaustauscherharz und dem Natriumsalz der 2-[4'-(p-Chlorbenzoyl)-phenoxy]-2-[methyl]-propionsäure, von denen es sich ableitet, sowie gegenüber deren mechanischen Mischung deutlich durch seine Überlegenheit auszeichnet, und zwar sowohl hinsichtlich der Verminderung des Triglyceridgehaltes im Blut im Fruchtzuckerversuch als auch hinsichtlich der Verminderung des Cholesteringehaltes im Blut. Im besonderen ist die Verminderung des Cholesteringehaltes im Blut nicht von irgendeiner Erhöhung des Wertes des Cholesteringehaltes in der Leber begleitet. Aus einer Betrachtung der vorstehenden Tabelle II geht nämlich hervor, dass der Cholesteringehalt in der Leber schon in der 7-ten Stunde vermindert ist und ohne weitere wesentliche Änderungen auf diesem Spiegel bleibt.

Besonders hervorzuheben ist der Synergismus der Wirkung des erfindungsgemässen Salzes die Verminderung des Cholesteringehaltes im Blut und in der Leber betreffend gegenüber der Summe der Wirkungen des Anionenaustauscherharzes und des Natriumsalzes der 2-[4'-(p-Chlorbenzoyl)-phenoxy]-2-[methyl]-propionsäure, von welchen es sich ableitet.

Eine vergleichbare Wirksamkeit hinsichtlich der Verminderung der Gehalte an Cholesterin und Triglyceriden im Blut wurde mit dem Salz eines mit 2 Gew.-% Divinylbenzol vernetzten Polystyrolharzes mit Aminogruppen in der quaternären Ammoniumchloridsalzform mit einer spezifischen Ionenaustauschkapazität von 3,5 Milliäquivalenten/g mit 2-[4'-(p-Chlorbenzoyl)-phenoxy]-2-[methyl]-propionsäure, bei welchem 1,99 % der Ionenaustauschkapazität des Anionenaustauscherharzes gesättigt (in das Salz überführt) waren (erfindungsgemässes Salz des Beispieles 3), und dem Salz eines mit Diäthylaminoäthylgruppen veräatherten vernetzten Dextranharzes mit einer spezifischen Ionenaustauschkapazität von 3,5 ± 0,5 Milliäquivalen/g mit 2-[4'-(p-Chlorbenzoyl)-phenoxy]-2-[methyl]-propionsäure, bei welchem 15 % der Ionenaustauschkapazität des Anionenaustauscherharzes in die Salzform überführt waren (erfindungsgemässes Salz des Beispieles 5), erhalten.

Ferner wurde die Wirksamkeit eines weiteren erfindungsgemässen Salzes sowie des als Vergleichsprodukt verwendeten entsprechenden Anionenaustauscherharzes und des ebenfalls als Vergleichsprodukt verwendeten Natriumsalzes der 2-[4'-(-Chlorbenzoyl)-phenoxy)-2-[methyl]-propionsäure in denselben Mengen, in welchen sie im erfindungsgemässen Salz enthalten waren, nach der im obigen Abschnitt C) beschriebenen Verfahrensweise bestimmt. Die Ergebnisse sind in der folgenden Tabelle III zusammengestellt.

Tabelle III

| Produkt | Tagesdosis | Verminderung, bezogen auf den Blindversuch | | |
|---------|-----------|:---:|:---:|:---:|
| | | Gesamtlipidgehalt | Gesamtcholesteringehalt | Triglyceridgehalt |
| Salz eines mit 2 Gew.-% Divinylbenzol vernetzten Polystyrolharzes mit Aminogruppen in der quaternären Ammoniumchloridsalzform (Dowex 1X2) mit 2-[4'-(p-Chlorbenzoyl)-phenoxy]-2-[methyl]-propionsäure, dessen Ionenaustauschkapazität zu 7,48% gesättigt ist [erfindungsgemässes Salz des Beispieles 4] | 2 × 650 mg/kg | − 43% | − 42% | − 56% |

Tabelle III

| Produkt | Tagesdosis | Verminderung, bezogen auf den Blindversuch | | |
|---|---|---|---|---|
| | | Gesamtlipid-gehalt | Gesamtchole-steringehalt | Triglycerid-gehalt |
| Das obige mit 2% Divinylbenzol vernetzte Polystyrolharz mit Aminogruppen in der quaternären Ammoniumchloridsalzform (Dowex 1X2) [Vergleichsprodukt] | 2×600 mg/kg | −21% | −30% | −27% |
| Natriumsalz der 2-[4'-(p-Chlorbenzoyl)-phenoxy]-2-[methyl]-propionsäure [Vergleichsprodukt] | 2×50 mg/kg | −39% | −36% | −46% |
| Mechanische Mischung aus 600 mg des obigen mit 2% Divinylbenzol vernetzten Polystyrolharzes mit Aminogruppen in der quaternären Ammoniumchloridsalzform (Dowex 1X2) und 50 mg des Natriumsalzes der 2-[4'-(p-Chlorbenzoyl)-phenoxy]-2-[methyl]-propionsäure [Vergleichsprodukt] | 2×650 mg/kg | −37% | −35% | −41% |

Auch aus der Tabelle III geht die Überlegenheit des erfindungsgemässen Salzes gegenüber dem verwendeten Anionenaustauscherharz und dem Natriumsalz der 2-[4-(p-Chlorbenzoyl)-phenoxy]-2-[methyl]-propionsäure, von welchen es sich ableitet, sowie deren mechanischen Mischung hervor.

Besonders hervorzuheben ist wiederum der Synergismus der Wirkung des erfindungsgemässen Salzes, vor allem die Verminderung des Triglyceridgehaltes betreffend, gegenüber der Summe der Wirkungen des Anionenaustauscherharzes und der 2-[4'-(p-Chlorbenzoyl)-phenoxy]-2-[methyl]-propionsäure, von welchen es sich ableitet.

Die Erfindung wird an Hand der folgenden Beispiele näher erläutert.

### Beispiel 1

Es wurden nach der unter den obigen Abschnitten I) und II) beschriebenen Verfahrensweise 6 g eines mit 2 Gew.-% Divinylbenzol vernetzten Polystyrolharzes mit Aminogruppen in der quaternären Ammoniumchloridsalzform (Dowex 1X2 [Produkt der Firma Dow Chemical Co.]) mit Teilchengrössen von 0,07 bis 0,15 mm [100-200 mesh] und mit einer spezifischen Ionenaustauschkapazität von 3,5 Milliäquivalenten/g gewaschen und mit 1 g (3,14 Milliäquivalenten) 2-[4'-(p-Chlorbenzoyl)-phenoxy]-2-[methyl]-propionsäure (dem Procetophen entsprechende freie Säure) in Lösung in 3,14 cm$^3$ einer n Natriumhydroxydlösung in ein Salz überführt. Die Säuremenge war so, dass 15 % der Ionenaustauschkapazität des Anionenaustauscherharzes gesättigt (in das Salz überführt) wurden.

### Beispiel 2

Beim Arbeiten nach derselben Verfahrensweise wie im Beispiel 1 und ausgehend von passenden Mengen derselben Reaktionsteilnehmer wurde ein Salz, bei welchem 2,38 % der Ionenaustauschkapazität des Anionenaustauscherharzes gesättigt (in das Salz überführt) war, erhalten.

### Beispiel 3

Es wurde nach der unter den obigen Abschnitten I) und II) beschriebenen Verfahrensweise ein fein vermahlenes [90 % Teilchen unter 0,074 mm {unter 200 mesh}] mit 2 Gew.-% Divinylbenzol vernetztes Polystyrolharz mit Aminogruppen in der quaternären Ammoniumchloridsalzform (Dowex 1—X—2) mit einer spezifischen Ionenaustauschkapazität von 3,5 Milliäquivalenten/g mit 2-[4'-(p-Chlorbenzoyl)-phenoxy]-2-[methyl]-propionsäure unter Verwendung von solchen Mengen in ein Salz überführt, dass 1,99 % der Ionenaustauschkapazität des Anionenaustauscherharzes gesättigt (in das Salz überführt) wurden.

### Beispiel 4

Es wurden nach der unter den obigen Abschnitten I) und II) beschriebenen Verfahrensweise 6 g eines mit 2 Gew.-% Divinylbenzol vernetzten Polystyrolharzes mit Aminogruppen in der quaternären Ammoniumchloridsalzform (Dowex 1X2) mit Teilchengrössen von 0,07 bis 0,15 mm (100-200 mesh) und mit einer spezifischen Ionenaustauschkapazität von 3,5 Milliäquivalenten/g mit 0,5 g 2-[4'-(p-Chlorbenzoyl)-phenoxy]-2-[methyl]-propionsäure unter Sättigung (Überführung in das Salz) von 7,48 % der Ionenaustauschkapazität des Anionenaustauscherharzes umgesetzt.

**0 012 804**

**Patentansprüche** (für die Vertragsstaaten : BE, CH, DE, FR, GB, NL, SE)

1. Salze eines zur dauernden Bindung an die Gallensäuren fähigen nicht toxischen aktivierten Anionenaustauscherharzes, nämlich eines mit etwa 2 Gew.-% Divinylbenzol vernetzten Polystyrolharz mit Aminogruppen in der quaternären Ammoniumchloridsalzform, mit 2-[4'-(p-Chlorbenzoyl)-phenoxy]-2-[methyl]-propionsäure, bei welchen die Ionenaustauschkapazität des Harzes zu 1,99 bis 15 % als Salz vorliegt.

2. Arzneimittel, gekennzeichnet durch einen Gehalt an einem oder mehreren Salzen nach Anspruch 1 als Wirkstoff beziehungsweise Wirkstoffen, gegebenenfalls zusammen mit einem oder mehreren pharmazeutischen Konfektionierungsmitteln.

**Patentanspruch** (für den Vertragsstaat AT)

Verfahren zur Herstellung von Salzen eines zur dauernden Bindung an die Gallensäuren fähigen nicht toxischen aktivierten Anionenaustauscherharzes mit 2-[4'-(p-Chlorbenzoyl)-phenoxy]-2-[methyl]-propionsäure, dadurch gekennzeichnet, daß man ein mit etwa 2 Gew.-% Divinylbenzol vernetztes Polystyrolharz mit Aminogruppen in der quaternären Ammoniumchloridsalzform mit 2-[4'-(p-Chlorbenzoyl)-phenoxy]-2-[methyl]-propionsäure umsetzt, wobei man die Ionenaustauschkapazität des Harzes zu 1,99 bis 15 % in Salze überführt.

**Claims** (for the Contracting States : BE, CH, DE, FR, GB, NL, SE)

1. Salts of a non-toxic activated anion exchange resin capable of being permanently bonded to the gallic acids, namely of a polystyrene resin crosslinked with about 2 % by weight of divinyl benzene and having amino groups in the quaternary ammonium chloride salt form, with 2-[4'-(p-chlorobenzoyl)-phenoxy]-2-[methyl]-propionic acid in which 1,99 to 15 % of the ion exchange capacity of the resin is present as a salt.

2. Medicines, characterized by a content of one or several salts according to claim 1 as an active ingredient or as active ingredients, respectively, optionally together with one or several pharmaceutical agents.

**Claim** (for the Contracting State AT)

A process for preparing salts of a non-toxic activated anion exchange resin with 2-[4'-(p-chlorobenzoyl)-phenoxy]-2-[methyl]-propionic acid capable of being permanently bonded to the gallic acids with 2-[4'-(p-chlorobenzoyl)-phenoxy]-2-[methyl]-propionic acid, characterized in that one reacts a polystyrene resin cross-linked with about 2 % by weight of divinyl benzene and having amino groups in the quaternary ammonium chloride salt form with 2-[4'-(p-chlorobenzoyl)-phenoxy]-2-[methyl]-propionic acid converting 1,99 to 15 % of the ion exchange capacity of the resin into salts.

**Revendications** (pour les Etats contractants : BE, CH, DE, FR, GB, NL, SE)

1. Sels d'une résine d'échange d'anions non toxique activée capable d'être liée en mode permanent aux acides biliaires, à savoir d'une résine de polystyrène réticulée avec de environ 2 % en poids de divinylbenzene et ayant groupes amino dans la forme de sels de chloride d'ammonium quaternaire, avec l'acide 2-[4'-(p-chlorobenzoyl)-phénoxy]-2-[méthyl]-propionique dans lesquels la capacité d'échange d'ions de la résine est présent à 1,99 à 15 % comme sel.

2. Médicaments, caractérisés d'une teneur d'un ou plusieurs sels selon la revendication 1 comme principe actif ou comme principes actifs, respectivement, éventuellement conjointement avec un ou plusieurs agents de confectionnements pharmaceutiques.

**Revendication** (pour l'Etat contractant AT)

Un procédé à la préparation de sels d'une résine d'échange d'anions non toxique activée capable d'être liés en mode permanent aux acides biliaires avec l'acide 2-[4'-(p-chlorobenzoyl)-phénoxy]-2-[méthyl]-propionique, caractérisé de ce qu'on fait réagir une résine de polystyrène réticulée avec environ 2 % en poids de divinylbenzene et ayant groupes amino dans la forme de sels de chloride d'ammonium quaternaire avec l'acide 2-[4'-(p-chlorobenzoyl)-phénoxy]-2-[méthyl]-propionique en convertissant la capacité d'échange d'ions de la résine à 1,99 à 15 % en sels.